# EUROPEAN PATENT APPLICATION

(11) **EP 1 304 132 A1**
(43) Date of publication of application: **23.04.2003**
(21) Application number: 02018421.4
(22) Date of filing: 16.08.2002
(51) Int. Cl.: A61M 16/18, F16L 29/04

(54) **Coupling system**

(30) Priority: 18.10.2001 SE 0103473
(71) Applicant: Siemens-Elema AB, 171 95 Solna 1 (SE)
(72) Inventor: Videbrink, Petter, 194 34 Upplands Väsby (SE)

(57) **Abstract**

A coupling system (2A) for transferring liquid anaesthetic from a bottle to a vaporiser, comprising a first part (2A), comprising a first valve with a first spring-loaded valve body (8A, 10) and a first counter body (12A), and a second valve part, comprising in the corresponding fashion, a second valve with a second spring-loaded valve body and a second counter body, the first part (2A) and the second part being interconnectable, whereby the first counter body (12A) is devised to act on the second spring-loaded valve body in an opening direction, and the second counter body is devised to act on the first spring-loaded valve body (8A, 10) in an opening direction to form a path of flow for the liquid anaesthetic. Leakage of liquid into atmosphere is reduced since the first spring-loaded valve body (8A, 10) and the first counter body (12A) are arranged so they, in a closed position, form an essentially flat first surface, and the second spring-loaded valve body and the second counter body are arranged in the corresponding way so they, in the closed position, form an essentially flat second surface.

## Description

The present invention relates to a coupling system according to the preamble to claim 1.

In inhalation anaesthesia, a gas mixture containing an anaesthetic is supplied to a patient via the airways. The anaesthetic is usually a liquid contained in a vaporiser, and a specific amount of liquid is vaporised, as needed, for delivery to the patient. The vaporiser holds a limited amount of liquid and may need refilling during ongoing anaesthesia.

When the vaporiser is refilled with liquid, keeping the anaesthetic from leaking into the surroundings and vaporising is important. There are two reasons for this. The anaesthetic agent's adverse impact on staff during ongoing surgery is one short-term effect. A long-term effect is that staff exposed to anaesthetic gases for a long time can develop cancer.

A plurality of coupling systems is known for replenishing liquid anaesthetic in a vaporiser. Some of the systems are made of two parts, one on the bottle holding the liquid and one on the vaporiser. Both these parts contain a moving, spring-loaded valve body and a counter body. When the parts are interconnected, the counter body in the respective part acts on the valve body in the other part to form a path of flow for the liquid. One common disadvantage with these coupling systems is the presence of residual anaesthetic when the parts are separated, and this liquid evaporates into atmosphere.

Known coupling systems are disclosed in e.g. WO92/12753.

In an alternative embodiment of the vaporiser, the bottle holding the liquid anaesthetic is used as a container, and, in principle, only the amount of liquid to be vaporised is transferred to the vaporiser. Even here, the same problem of residual liquid is found when the bottle is detached from the vaporiser.

One objective of the present invention is to achieve a coupling system for transferring liquid anaesthetic from a bottle to a vaporiser, said system at least partially solving some of the problems encountered in the prior art technology.

This objective is achieved according to the invention when the coupling system according to the above is devised as is evident from the characterising part of claim 1.

Advantageous embodiments are evident from the subordinate claims of claim 1.

Fluid spilling is reduced to a minimum when the coupling system is devised in two parts so the spring-loaded valve body and the counter body in the respective part form an essentially flat surface. In principle, there is no space between parts in which fluid can accumulate.

Even if the respective valve body is simultaneously actuated in the opening direction, the parts ate devised so the passage for the flow of liquid (and gas) opens in one of the parts first. The vaporiser part should open first and close last when the parts are interconnected or detached during sequential opening of the flow passage.

An additional gasket arranged on one of the parts to seal against the other part before the valve bodies are affected also reduces the risk of gas leakage from the coupling system.

One of the parts can be permanently mounted on the vaporiser, whereas the other part can be permanently mounted on a bottle or consist of an adapter suitable for mounting on a bottle.

One embodiment of the coupling system according to the invention is described below in greater detail, referring to the figures.
FIG. 1 is a schematic depiction of a first part in the coupling system, and
FIG. 2 is a schematic depiction of a second part in the coupling system.

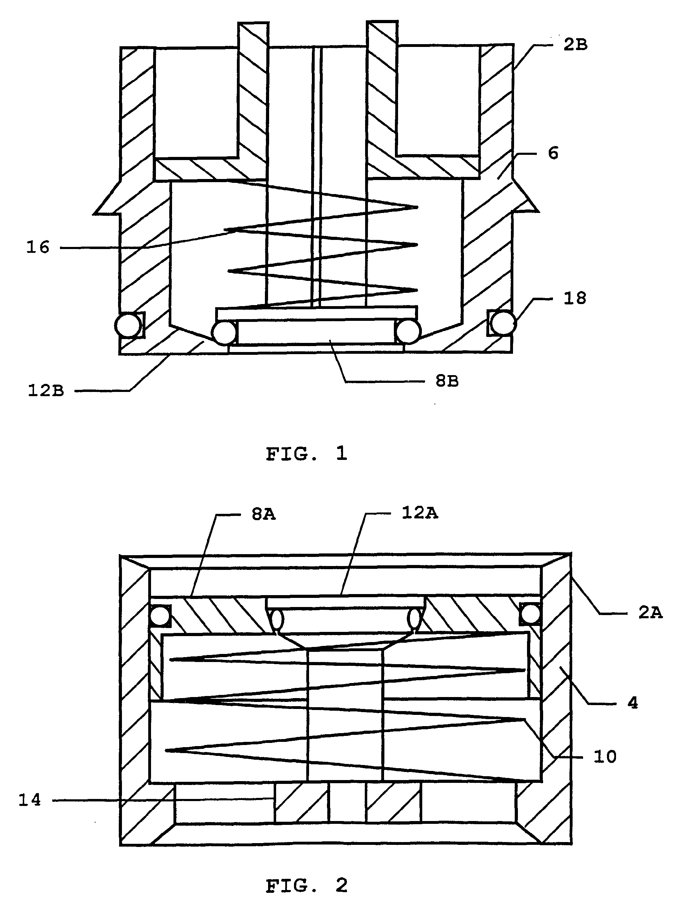

Reference is simultaneously made below to both FIG. 1 and FIG. 2. They illustrate the two parts in the embodiment of the coupling system according to the invention.

The first part 2A is connectable to a second part 2B. In this embodiment, the first part 2A is connected to a vaporiser (not shown), and the second part 2B is connected to or is part of a bottle (not shown) containing liquid anaesthetic.

The first part 2A has a cylindrical casing 4 with an internal diameter tailored to the external diameter of a casing 6 for the second part 2B.

The first part 2A has a first valve body 8A that is biased against a closed position by a first spring 10. In principle, the first valve body 8A has a circular cross-section surrounding a centrally located first counter body 12A. The first counter body 12A rests on a part 14 of the casing 4. The first valve body 8A and the first counter body 12A are devised to form an essentially flat first surface.

In the corresponding manner, the second part 2B has a valve body 8B biased in a closed position by a second spring 16 and a second counter body 12B. The second counter body 12B consists of the outer part of the casing 6. The second valve body 8B and the second counter body 12B are also devised to form an essentially flat second surface.

In use, the parts 2A, 2B are conjoined by inserting the second part 2B into the first part 2A. Before the flat surfaces touch, a gasket 18 in the second part 2B seals against the inner surface of the first part's 2A casing 4 and ensures that no liquid or gas is able to leak out of the coupling system once a passage for liquid is established.

When the parts 2A, 2B, are pushed further together, the first counter body 12A begins to press the second valve body 8B against the closing force of the second spring 16 and opens a passage for liquid from the bottle. At the same time, the second counter body 12B presses the first valve body 8A against the closing force of the first spring 10 and opens a passage for liquid into the vaporiser.

The reverse applies when the parts 2A, 2B are detached, i.e. the valve bodies 8A, 8B are forced against a common plane by the counter bodies 12A, 12B. There is then no space capable of harbouring any residual liquid, and leakage into atmosphere is minimal.

The procedure described above provides essentially simultaneous opening of flow paths for liquid in both parts 2A, 2B. The parts can naturally be devised for sequential opening of the flow passages during simultaneous activation of the valve bodies 8A, 8B. All that is necessary is for e.g. the first valve body 8A to have a longer opening path than the second valve body 8B.

## Claims

1. A coupling system (2A, 2B) for transferring liquid anaesthetic from a bottle to a vaporiser, comprising a first part (2A), comprising a first valve with a first spring-loaded valve body (8A, 10) and a first counter body (12A), and a second valve part (2B), comprising a second valve with a second spring-loaded valve body (8B, 16) and a second counter body (12B), the first part (2A) and the second part (2B) being interconnectable, whereby the first counter body (12A) is devised to act on the second spring-loaded valve body (8B, 16) in an opening direction, and the second counter body is devised to act on the first spring-loaded valve body (8A, 10) in an opening direction to form a path of flow for the liquid anaesthetic, **characterised in that** the first spring-loaded valve body (8A, 10) and the first counter body (12A) in a closed position are arranged to form an essential flat first surface, and the second spring-loaded valve body (8B, 16) and the second counter body (12B) in a closed position are arranged to form an essentially flat second surface.

2. A coupling system according to claim 1, **characterised in that** the second part (2B) also contains a gasket (18) arranged to interact with the first part (2A) during interconnection before the first surface and second surface coincide.

3. A coupling system according to claim 1 or 2, **characterised in that** the second part (2B) consists of an adapter devised to be arranged on the bottle.
